(19) Europäisches Patentamt

European Patent Office

Office européen des brevets

(11) Veröffentlichungsnummer: **0 226 069**
**A1**

(12) **EUROPÄISCHE PATENTANMELDUNG**

(21) Anmeldenummer: **86116139.6**

(22) Anmeldetag: **21.11.86**

(51) Int. Cl.⁴: **C 12 N 15/00**
**C 07 H 21/04, C 12 P 21/00**
**G 01 N 33/569, G 01 N 33/5-77**
**//A61K39/395**

(30) Priorität: **28.11.85 DE 3542024**
**21.12.85 DE 3545576**

(43) Veröffentlichungstag der Anmeldung:
**24.06.87 Patentblatt 87/26**

(84) Benannte Vertragsstaaten:
**AT BE CH DE ES FR GB IT LI LU NL SE**

(71) Anmelder: **BEHRINGWERKE Aktiengesellschaft**
**Postfach 1140**
**D-3550 Marburg 1(DE)**

(72) Erfinder: **Szöts, Hannelore**
**Kiltrachinger Strasse 18**
**D-8031 Gilching(DE)**

(72) Erfinder: **Weiss, Eliabeth, Dr.**
**Dauthendey Strasse 11**
**D-8000 München 70(DE)**

(72) Erfinder: **Dörner, Christa**
**Massenhausener Strasse 4**
**D-8056 Neufahrn(DE)**

(72) Erfinder: **Lang, Margot**
**Geyerstrasse 12**
**D-8000 München 5(DE)**

(72) Erfinder: **Meo, Tomaso, Dr.**
**18 Rue Quincampoix**
**F-75004 Paris(FR)**

(72) Erfinder: **Riethmüller, Gert, Prof. Dr.**
**Hermann-Vogel-Strasse 24**
**D-8000 München(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) HLA-B 27, dafür codierende DNA und ihre Verwendung.

㊿ Aus einer genomischen und einer cDNA-Bibliothek wurden die genomische DNA und die cDNA für HLA-B 27 gewonnen, die einerseits als Diagnostikum und andererseits als Ausgangsmaterial zur gentechnischen Herstellung von HLA-B 27 dienen können. Das so erhaltene HLA-B 27 kann seinerseits als Diagnostikum sowie zur Herstellung von polyklonalen und monoklonalen Antikörpern dienen.

EP 0 226 069 A1

1

0226069

BEHRINGWERKE AKTIENGESELLSCHAFT    HOE 85/B 023 K    Dr.KL/mü

## HLA-B 27, dafür codierende DNA und ihre Verwendung

HLA-B 27 gehört als humanes Antigen der Klasse I zu den humanen Histokompatibilitäts-Antigenen. Erhöhtes Auftreten von HLA-B 27 gilt als hoher Risikofaktor für eine Gruppe rheumatischer Erkrankungen, unter denen Ankylosis spondylitis im Vordergrund steht (Thomson, G., Theoret. Pop. Biol. 20 (1981) 168 - 208).

Mit Hilfe einer geeigneten Sonde (aus der 3'-untranslatierten Region (Cosman, D., et al., Nature 295 (1982) 73 - 76) einer HLA-homozygoten B-Zellinie (R.A. Gatti et al., Tissue Antigens 13 (1979) 35 - 44)) konnte die cDNA und die genomische DNA für HLA-B 27 isoliert, identifiziert und sequenziert werden. Die Nukleotidfolge ist im Anhang wiedergegeben. Anhang I zeigt die genomische DNA (codierender Strang) und über den Exons die Aminosäuresequenz. Anhang II zeigt die Nucleotidsequenz der cDNA, sie entspricht den Exons 2 bis 7 der genomischen DNA.

Auf der Grundlage dieser ermittelten DNA-Sequenzen kann der Fachmann nach üblichen Methoden der chemischen Gensynthese, beispielsweise nach der Phosphitmethode, die Genstruktur reproduzieren. Außerdem ist es möglich, in Kenntnis dieser Nukleotidsequenzen Sonden zu synthetisieren und damit aus einer geeigneten cDNA-Bank oder genomischen Bank in bekannter Weise die cDNA oder die genomische Sequenz zu gewinnen. Im Falle der chemischen Synthese kann von den natürlichen Schnittstellen (Anhang III) Gebrauch gemacht werden.

Die derart gewonnene DNA kann in bekannter Weise in einen Vektor eingebaut werden, beispielsweise in einen der bekannten handelsüblichen E. coli-Vektoren wie pBR 322 oder pBR 325 oder der pUC-Vectoren wie pUC 8, 9, 12, 13, 18 oder 19, und in einem geeigneten Wirt, beispielsweise E. coli, amplifiziert werden. Die reisolierte DNA kann dann ihrerseits als Gensonde ("full length probe") für diagno-

stische Zwecke dienen. Die Erfindung betrifft somit auch die Verwendung der DNA als Diagnostikum bzw. zur Herstellung eines diagnostischen Mittels bzw. ein Diagnostizierverfahren, in welchem isoliertes humanes genetisches Material auf die Anwesenheit eines - gegebenenfalls mutierten - für HLA-B 27 codierenden Gens oder die entsprechende mRNA untersucht wird.

Die Erfindung betrifft weiterhin die Verwendung der DNA zur gentechnologischen Herstellung von HLA-B 27, wobei einerseits in an sich bekannter Weise (EP-A 0 090 505) die codierende DNA in einen Expressionsvektor eingebaut und dieser in eine geeignete Wirtszelle eingebracht oder andererseits die genomische DNA direkt in eine eukaryotische Wirtszelle eingeführt, jeweils dort das Polypeptid exprimiert und nach ebenfalls an sich bekannten Methoden isoliert wird. Anstelle der isolierten genomischen DNA kann auch ein 6,5 kb großes EcoRI-Fragment oder auch ein entsprechender Klon aus der verwendeten Genbank eingesetzt werden. Weiterhin können die für eukaryotische Wirtszellen erprobten Vektorsysteme angewendet werden, insbesondere virale Vektoren oder in Hefezellen dafür speziell erarbeitete Vektoren.

Das erfindungsgemäß gewonnene HLA-B-27 kann als Diagnostikum dienen, beispielsweise zur Feststellung, ob ein Humanserum Antikörper gegen HLA-B 27 enthält. Die Erfindung betrifft somit auch ein Diagnostizierverfahren zur Feststellung von Antikörpern gegen HLA-B 27, das dadurch gekennzeichnet ist, daß man ein diese Antikörper möglicherweise enthaltendes Humanserum mit HLA-B 27 in Kontakt bringt.

Das erfindungsgemäß erhaltene HLA-B 27 kann außerdem als Antigen zur Erzeugung von HLA-B 27-spezifischen Antikörpern eingesetzt werden. Hierzu immunisiert man in üblicher Weise Versuchstiere wie Mäuse, Kaninchen, Schafe oder Ziegen und gewinnt in an sich bekannter Weise das die gebildeten Antikörper enthaltende Serum.

Zur Herstellung von monoklonalen Antikörpern wird das Antigen in folgender Weise eingesetzt: Man fusioniert eine Antikörper gegen HLA-B 27 produzierende Zelle, beispielsweise eine Mäusezelle, mit einer Myelomazelle und kultiviert die fusionierten Zellhybride in bekannter Weise (z.B. nach den in den US-PSS 4 196 265, 4 474 893 oder 4 451 570 beschriebenen Verfahren).

Die so erhaltenen polyklonalen und monoklonalen Antikörper können in an sich bekannter Weise für Diagnostika eingesetzt werden, wobei die üblichen Methoden Verwendung finden. So kann beispielsweise humanes Serum mit diesen Antikörpern in Kontakt gebracht werden und so die Anwesenheit erhöhter Mengen an HLA-B 27 festgestellt werden. So können humane kernhaltige Zellen, bevorzugt Lymphozyten, in einem üblichen Zytotoxyzitätstest oder einem Bindungstest unter Ausnutzung der Antigen-Antikörper-Reaktion mit Hilfe eines Marker-Enzyms, bevorzugt ein Fluoreszenzfarbstoff oder ein Enzym, zum Nachweis von HLA-B 27 eingesetzt werden.

Erfindungsgemäß ist somit eine Vielfalt von diagnostischen Möglichkeiten gegeben, HLA-B 27 und das dafür codierende Gen festzustellen. Die Erfindung bereichert somit die diagnostischen Möglichkeiten auf dem Gebiet der humanen Antigene der Klasse I.

Beispiel 1

Mit Hilfe des Vektors pTCF (Grosveld et al., Nucl. Acids Res. 10 (1982) 6715; Gene 13, (1981) 227) wurde aus peripheren weißen Blutzellen eines gesunden Spenders vom HLA-Typ HLA-A2/2, -B5/27, -Cw2/3 eine genomische Bank angelegt. Diese Cosmidbank wurde mit einer HLA-B-Locus-spezifischen cDNA-Sonde aus der 3' untranslatierten Region des HLA-B 8-Gens sondiert. Die DNA-Sequenzanalyse von 3'-Bgl II-Fragmenten, die das ganze HLA-B 27 Gen enthalten und in dem Vektor pUC 13 subcloniert waren, erfolgte im wesentlichen nach der Didesoxy-Methode nach Sanger und nur teilweise, um zweifelhafte Nucleotide zu identifizieren, durch die chemische Sequenzierungsmethode von Maxam & Gilbert.

Maus-Thymidin-Kinase-negative Zellen (tk⁻-Zellen), LD 1 ($H-2^k$) wurden mit der Cosmid-DNA und dem Herpes-tk-Gen, enthalten im Vektor pOPF (Grosveld et al., Nucl. Acids Res. 10, 6715) mittels der Calciumphosphat-Transfertechnik transfiziert (Wigler et al., Proc. Natl. Acad. Sci. USA 76 (1979) 1373). Populationen transformierter Zellen wurden mit Hilfe der Hypoxanthin-Aminopterin-Thymidin-Selektion (HAT) selektiert und stabile Ltk⁺ transformierte Clone identifiziert. Die humane lymphoblastoide Zellinie LG-2 (HLA-A 2/2; B 27/27; Cw1/1) wurde als positive Kontrolle für die HLA-B 27-Expression gewählt.

Beispiel 2

Zur Gewinnung polyklonaler Antikörper werden Versuchstiere, z.B. Kaninchen, mit dem erfindungsgemäß hergestellten HLA-B 27 immunisiert. Dazu kann das Antigen nach bekannten Verfahren an einen Träger, z.B. ein Protein, gekoppelt werden, um die Immunantwort zu verstärken. Das Antigen, als solches oder an das Trägerprotein gebunden, kann zur Erzielung einer besseren Immunantwort mit Hilfe eines Adjuvans, z.B. eines Silikates, eines Aluminates oder einer ölhaltigen Adjuvans wie Freundsches Adjuvans, Versuchstieren injiziert werden, z.B. intravenös, intramuskulär oder subcutan.

Beispiel 3

a) Zur Herstellung von Diagnostika für den Zytotoxizitätstest werden die erfindungsgemäß erhaltenen polyklonalen oder monoklonalen Antikörper mit einer schutzkolloidhaltigen Pufferlösung so verdünnt, daß sie im NIH-Zytotoxizitätstest ein optimales Reaktionsverhalten ergeben.

b) Zur Herstellung von Fertigreagenzien für den Zytotoxizitätstest werden die erfindungsgemäß erhaltenen polyklonalen oder monoklonalen Antikörper adsorptiv auf Kunststofftestplatten, z.B. Mikrotestplatten nach Terasaki, gebunden.

Beispiel 4

Zur Herstellung von Diagnostika für den Bindungstest werden die erfindungsgemäß erhaltenen polyklonalen oder monoklonalen Antikörper mit einem Fluoreszenzfarbstoff, z.B. Fluoresceinisothiocyanat (FITC) oder Tetramethylrhodaminisothiocyanat (TRITC), oder mit einem Enzym, z.B. Peroxidase oder alkalische Phosphatase, gekoppelt.

Beispiel 5

Zur Herstellung von Diagnostika zum Nachweis von HLA-B 27 in Humanserum werden die erfindungsgemäß erhaltenen polyklonalen oder monoklonalen Antikörper adsorptiv auf Kunststofftestplatten, z.B. Mikrotitrationsplatten, gebunden. Mit Hilfe von nach Beispiel 3 hergestellten Enzym-gekoppelten polyklonalen oder monoklonalen Antikörpern wird ein Testsystem, z.B. nach dem ELISA-Prinzip, hergestellt.

Beispiel 6

Zur Herstellung von Diagnostika zum Nachweis von Antikörpern gegen HLA-B 27 in humanen oder tierischen Antisera wird das erfindungsgemäß hergestellte HLA-B 27, z.B. adsorptiv, an die Oberfläche von Kunststofftestplatten gebunden und der nachzuweisende Antikörper gegen HLA-B 27 durch einen z.B. Enzym-gekoppelten Antikörper gegen humanes bzw. tierisches Immunglobulin oder IgG, z.B. nach dem ELISA-Prinzip, bestimmt.

Anhang I: Nucleotidsequenz I (DNA I) Anhang II: cDNA-Sequenz (codierender Strang)

GGC TCC CAC TCC ATG AGG TAT TTC CAC ACC TCC GTG TCC CGG CCC GGC CGC GGG GAG CCC

CGC TTC ATC ACC GTG GGC TAC GTG GAC GAC ACG CTG TTC GTG AGG TTC GAC AGC GAC GCC

GCG AGT CCG AGA GAG GAG CCG CGG GCG CCG TGG ATA GAG CAG GAG GGG CCG GAG TAT TGG

GAC CGG GAG ACA CAG ATC TGC AAG GCC AAG GCA CAG ACT GAC CGA GAG GAC CTG CGG ACC

CTG CTC CGC TAC TAC AAC CAG AGC GAG GCC GGG TCT CAC ACC CTC CAG AAT ATG TAT GGC

TGC GAC GTG GGG CCG GAC GGG CGC CTC CTC CGC GGG TAC CAC CAG GAC GCC TAC GAC GGC

AAG GAT TAC ATC GCC CTG AAC GAG GAC CTG AGC TCC TGG ACC GCC GCG GAC ACG GCG GCT

CAG ATC ACC CAG CGC AAG TGG GAG GCG GCC CGT GTG GCG GAG CAG CTG AGA GCC TAC CTG

GAG GGC GAG TGC GTG GAG TGG CTC CGC AGA TAC CTG GAG AAC GGG AAG GAG ACG CTG CAG

CGC GTG GAC CCC CCA AAG ACA CAC GTG ACC CAC CAC CCC ATC TCT GAC CAT GAG GCC ACC

CTG AGG TGC TGG GCC CTG GGC TTC TAC CCT GCG GAG ATC ACA CTG ACC TGG CAG CGG GAT

GGC GAG GAC CAA ACT CAG GAC ACT GAG CTT GTG GAG ACC AGA CCA GCA GGA GAT AGA ACC

TTC CAG AAG TGG GCA GCT GTG GTG GTG CCT TCT GAA GAA GAG CAG AGA TAC ACA TGC CAT

GTA CAG CAT GAG GGG CTG CCG AAG CCC CTC ACC CTG AGA TGG GAG CCG TCT TCC CAG TCC

ACC GTC CCC ATC GTG GGC ATT GTT GCT GGC CTG GCT GTC CTA GCA GTT GTG GTC ATC GGA

GCT GTG GTC GCT GCT GTG ATG TGT AGG AGG AAG AGC TCA GGT GGA AAA GGA GGG AGC TAC

TCT CAG GCT GCG TGC AGC GAC AGT GCC CAG GGC TCT GAT GTG TCT CTC ACA GCT TGA AAA

GCC TGA

Anhang III: Schnittstellen in der cDNA

Endonuclease       Schnittstelle nach
           Nucleotid Nr. / Triplett Nr.
              (codierender Strang)

| Endonuclease | Nucleotid Nr. / Triplett Nr. |
|---|---|
| Taq I | 107 / 36 |
| Hinf I | 123 / 41 |
| Bgl II | 194 / 65 |
| Bgl II | 318 / 106 |
| Kpn I | 339 / 113 |
| Sac I | 394 / 132 |
| Sau 3 A | 422 / 141 |
| Bgl I | 454 / 152 |
| Pvu II | 465 / 155 |
| Pst I | 539 / 180 |
| Sau 3 A | 635 / 212 |
| Pvu II | 736 / 246 |
| Sac I | 937 / 313 |

## Patentansprüche

1. Genomische DNA und cDNA, codierend für HLA-B 27, mit der Nucleotidsequenz I und II (Anhang).

2. Verfahren zur Herstellung von HLA-B 27, dadurch gekennzeichnet, daß man die DNA der Nukleotidsequenz I oder II (Anhang) in einer Wirtszelle exprimiert.

3. Diagnostikum, gekennzeichnet durch einen Gehalt an der DNA I oder II oder einem charakteristischen Teil dieser DNA-Sequenzen.

4. Diagnostikum, gekennzeichnet durch einen Gehalt an HLA-B 27.

5. Diagnostikum, gekennzeichnet durch einen Gehalt an einem Antikörper gegen HLA-B 27.

6. Verfahren zur Herstellung von Antikörpern gegen HLA-B 27, dadurch gekennzeichnet, daß man Versuchstiere mit HLA-B 27 immunisiert und das die Antikörper enthaltende Serum gewinnt.

7. Verfahren zur Herstellung von monoklonalen Antikörpern gegen HLA-B 27, dadurch gekennzeichnet, daß man eine Antikörper gegen HLA-B 27 produzierende Zelle mit einer Myelomazelle fusioniert.

8. Diagnostizierverfahren, dadurch gekennzeichnet, daß man humanes genetisches Material mit einem Diagnostikum gemäß Anspruch 3 in Kontakt bringt.

9. Diagnostizierverfahren, dadurch gekennzeichnet, daß man humanes Serum mit einem Diagnostikum nach Anspruch 4 in Kontakt bringt.

10. Diagnostizierverfahren, dadurch gekennzeichnet, daß
    man humanes Serum mit einem Diagnostikum nach Anspruch 5
    in Kontakt bringt.

## EUROPÄISCHER TEILRECHERCHENBERICHT,
der nach Regel 45 des Europäischen Patentübereinkommens für das weitere Verfahren als
europäischer Recherchenbericht gilt

Europäisches
Patentamt

**0226069**
Nummer der Anmeldung

## EINSCHLÄGIGE DOKUMENTE

EP 86116139.6

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | CHEMICAL ABSTRACTS, Band 104, Nr. 21, 26. Mai 1986, Columbus, Ohio, USA<br><br>H.SZOTS et al. "Complete sequence of HLA-B 27 cDNA identified through the characterization of structural markers unique to the HLA-A,-B and -C alletic series"<br>Seite 144, rechte Spalte, Zusammenfassung-Nr. 180 964q<br><br>& Proc. Natl. Acad. Sci. U.S.A. 1986, 83(5), 1428-32<br><br>-- | 1,2 | C 12 N 15/00<br>C 07 H 21/04<br>C 12 F 21/00<br>G 01 N 33/569<br>G 01 N 33/577<br>//A 61 K 39/395 |
| X | WO - A1 - 82/02 060 (YALE UNIVERSITY)<br><br>* Ansprüche 1,9,10,13-15,18,19, 22-24 *<br><br>-- | 1,2 | |

### RECHERCHIERTE SACHGEBIETE (Int. Cl.4)

C 12 N
C 07 H
C 12 P
G 01 N
A 61 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7-10

Unvollständig recherchierte Patentansprüche: —

Nicht recherchierte Patentansprüche: 6

Grund für die Beschränkung der Recherche:

Artikel 52(4) EPÜ

| Recherchenort | Abschlußdatum der Recherche | Prüfer |
|---|---|---|
| WIEN | 03-03-1987 | WOLF |

**KATEGORIE DER GENANNTEN DOKUMENTEN**
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches
Patentamt
**EUROPÄISCHER TEILRECHERCHENBERICHT**

EP 86116139.6

| | EINSCHLÄGIGE DOKUMENTE | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, Band 101, Nr. 23, 3. Dezember 1984, Columbus, Ohio, USA<br><br>F.C.GRUMET et al. "An HLA-B locus probe clarifies endonucleare polymorphism of major histocompatibility complex class I genes" Seite 140, rechte Spalte, Zusammenfassung-Nr. 205 148e<br><br>& Mol. Biol. Med. 1983, 1(5),501-9<br><br>-- | 1-4 | |
| D,A | NATURE, Band 295, Nr. 5844, 7.-13. Jänner 1982, New York, London<br><br>D.COSMAN et al. "Three classes of mouse H-2 messenger RNA distinguished by analysis of cDNA clones"<br>Seiten 73-76<br><br>* Gesamt *<br><br>-- | 1,2 | RECHERCHIERTE SACHGEBIETE (Int. Cl.4) |
| A | US - A - 4 265 873 (SHEEHY et al.)<br>* Zusammenfassung *<br><br>-- | 5,7-10 | |
| A | US - A - 4 471 056 (GRUMET et al.)<br>* Zusammenfassung *<br><br>-- | 5,7-10 | |
| A | US - A - 4 492 760 (DEFREITAS)<br>* Zusammenfassung *<br><br>---- | 5,7-10 | |